# EUROPÄISCHE PATENTSCHRIFT

(11) **EP 0 481 317 B1**
(45) Veröffentlichungstag und Bekanntmachung des Hinweises auf die Patenterteilung: **09.08.1995**
(21) Anmeldenummer: 91117029.8
(22) Anmeldetag: 07.10.1991
(51) Int. Cl.: C07C 17/00, C07C 25/22, C07C 25/13, C07C 43/225

(54) **Verfahren zum Dechlorieren und/oder Debromieren von Fluor und Chlor und/oder Brom enthaltenden aromatischen Verbindungen**
Method for dechlorination and/or debromination of aromatic compounds containing fluorine with chlorine and/or bromine
Procédé pour déchloruration et/ou débromination des composés aromatiques contenant du fluor et du chlore et/ou du brome

(30) Priorität: 18.10.1990 DE 4033097
(43) Veröffentlichungstag der Anmeldung: 22.04.1992
(73) Patentinhaber: BAYER AG, 51368 Leverkusen (DE)
(72) Erfinder: Bielefeldt, Dietmar, Dr., W-4030 Ratingen 6 (DE); Marhold, Albrecht, Dr., W-5090 Leverkusen 1 (DE)

(56) Entgegenhaltungen:
- EP-A- 0 301 343
- WO-A-90/11987
- DE-A- 2 928 745
- FR-A- 2 615 185
- US-A- 4 925 998

## Beschreibung

Die vorliegende Erfindung betrifft ein technisch besonders vorteilhaftes und selektives Verfahren zum Dechlorieren und/oder Debromieren von Fluor und Chlor und/oder Brom enthaltenden aromatischen Verbindungen.

Es ist bekannt, daß man mindestens 3 Fluoratome enthaltende aromatische Verbindungen erhalten kann, indem man eine mindestens drei Fluoratome und mindestens ein weiteres nicht-Fluor-Halogenatom enthaltende aromatische Verbindung mit einer Säure und einem fein verteilten Metallpulver behandelt (siehe GB-PS 1 067 412). Setzt man in dieses Verfahren 1,3,5-Trichlor-2,4,6-trifluorbenzol, Essigsäure und Zinkpulver ein, so werden aus dem Reaktionsprodukt 91 % des Einsatzmaterials wiedergewonnen und nur 8 % 1,3-Dichlor-2,4,6-trifluorbenzol erhalten (siehe Beispiel 6 der GB-PS 1 067 412).

Weiterhin ist aus der DE-OS 3 909 213, Beispiel 24, die Sumpfphasen-Hydrierung von 2,6-Dichlor-4-trifluormethyl-3,5-difluortoluol mit Wasserstoff in Gegenwart von Palladium-auf-Kohle als Katalysator bekannt, wobei man in 78 %iger Ausbeute das entsprechende chlorfreie Produkt 4-Trifluormethyl-3,5-difluortoluol erhält.

Schließlich ist aus der DE-OS 2 928 745, Beispiel 1, bekannt, daß man in der Sumpfphase ein Gemisch von isomeren 1-Chlor-trifluormethylnaphthalinen in Methanol mit Raney-Nickel als Katalysator in 62,6 %-iger Ausbeute in Trifluormethylnaphthalin überführen kann.

Bei allen diesen Verfahren ist die erzielbare Ausbeute und Selektivität an dechlorierten Produkten noch unbefriedigend.

Es wurde nun ein Verfahren zur selektiven Dechlorierung und/oder Debromierung von Fluor und Chlor und/oder Brom enthaltenden aromatischen Verbindungen mit Wasserstoff in Gegenwart von Katalysatoren gefunden, das dadurch gekennzeichnet ist, daß sich der Katalysator in stationärer Phase und die Fluor und Chlor und/oder Brom enthaltende aromatische Verbindung in mobiler Phase befinden.

In das erfindungsgemäße Verfahren können z.B. Fluor und Chlor und/oder Brom enthaltende aromatische Verbindungen der Formel (I) eingesetzt werden 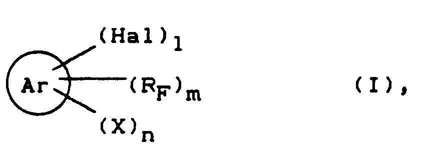
in der
- Ar: für einen aromatischen Rest mit 6 bis 10 C-Atomen,
- Hal: unabhängig voneinander für Chlor oder Brom,
- R_{F}: unabhängig voneinander für Fluor oder einen Fluor enthaltenden Rest mit 1 bis 4 C-Atomen und 1 bis 6 Fluoratomen,
- X: für C₁- bis C₄-Alkyl, fluoriertes C₁- bis C₄-Alkyl, C₁- bis C₄-Alkoxy, CHO, CH₂OH, CN, COO-C₁- bis C₄-Alkyl, NH₂, CH₂NH₂ oder NO₂,
- l: für 1, 2, 3 oder 4,
- m: für 1, 2, 3 oder 4 und
- n: für Null oder 1 stehen,
wobei l + m + n mindestens 2 und höchstens der Anzahl möglicher Valenzen an Ar entspricht.

Ar steht vorzugsweise für Phenyl oder Naphthyl, insbesondere für Phenyl.

Hal steht vorzugsweise für Chlor.

R_{F} steht vorzugsweise für Fluor, CF₃, CHF₂, CH₂F, CF₂CF₃, CH₂CF₃, CH₂CHF₂, CH₂CH₂F, OCF₃, OCF₂H, OCH₂F, OCF₂CF₂H, OCF₂CF₃ oder N(CF₃)₂.

Liegen 2 oder mehr Reste R_{F} vor, so können diese gleich oder verschieden sein. 2 Reste R_{F} können auch gemeinsam für -O-CF₂-CF₂-O-, -O-CF₂-CH₂-O-, -O-CF₂-CHF-O- oder -O-CF₂-O- stehen.

X steht vorzugsweise für CH₃, CF₃, OCH₃, OCH₂CH₃, CHO oder CN.

l steht vorzugsweise für 1 oder 2, m vorzugsweise für 1, 2 oder 3.

Falls Ar für Phenyl steht, ist l + m + n vorzugsweise 2, 3, 4, 5 oder 6, insbesondere 3, 4 oder 5. Falls Ar für Naphthyl steht, ist l + m + n vorzugsweise 2, 3 oder 4.

Wenn Ar für Phenyl steht ist es außerdem bevorzugt, daß wenigstens ein Rest R_{F} und wenigstens ein Rest Hal in ortho- oder para-Stellung zueinanderstehen.

Bezogen auf ein Äquivalent Chlor- und Bromatome, die in der jeweils eingesetzten Fluor und Chlor und/oder Brom enthaltenden aromatischen Verbindung enthalten sind, können in das erfindungsgemäße Verfahren beispielsweise 1 bis 50 Mol Wasserstoff eingesetzt werden. Vorzugsweise beträgt diese Menge 2 bis 20 Mol.

Damit sich der Katalysator während des erfindungsgemäßen Verfahrens in stationärer Phase befinden kann, liegt er am zweckmäßigsten in stückiger Form vor. Geeignet sind beispielsweise Trägerkatalysatoren, die Übergangsmetalle als katalytisch aktive Komponente enthalten. Als Übergangsmetalle sind Palladium, Platin, Nickel, Cobalt, Ruthenium, Rhenium und Rhodium bevorzugt, insbesondere Palladium und Platin. Beispiele für Trägermaterialien sind Aktivkohlen, Aluminiumoxide, Siliciumdioxide, Bariumsulfat, Spinelle, Silikate und Titandioxid. Bevorzugt sind Aktivkohlen und Lithium-Aluminium-Spinelle. Die Katalysatoren können pro Liter beispielsweise 0,1 bis 50 g Übergangsmetalle enthalten. Vorzugsweise beträgt dieser Gehalt 2 bis 20 g/l.

Geeignete Drucke für das erfindungsgemäße Verfahren sind beispielsweise solche im Bereich 0,1 bis 20 bar.

Das erfindungsgemäße Verfahren kann man beispielsweise bei Temperaturen über 100°C, bevorzugt bei 120°C bis 400°C, insbesondere bei 150 bis 350°C, durchführen.

Es ist ein wesentliches Merkmal der vorliegenden Erfindung, daß sich der Katalysator in stationärer Phase und die Fluor und Chlor und/oder Brom enthaltende aromatische Verbindung (= Edukt) in mobiler Phase, gegebenenfalls zusammen mit dem Wasserstoff und/oder flüssigen oder gasförmigen inerten Stoffen, befindet. Beispielsweise kann man in einem Reaktionsraum, z.B. einem röhrenförmigen Reaktor, den Katalysator fest anordnen und den Wasserstoff im Gemisch mit dem Edukt gasförmig durch das Katalysatorbett leiten. Die Gasphase kann dabei auch Inerte enthalten, z.B. Stickstoff, Edelgase und/oder Dämpfe inerter Lösungsmittel, z.B. Dämpfe von Toluol, Xylol, Methylcyclohexan, Ligroin und/oder Petrolether.

Wenn die Gasphase Inerte enthält, handelt es sich dabei vorzugsweise um Lösungsmitteldämpfe. Man kann auch mit einer Gasphase arbeiten, die nur das Edukt und Wasserstoff enthält.

Man kann auch in der sogenannten Rieselphase arbeiten, d.h. über einen fest angeordneten Katalysator Wasserstoff gasförmig und das Edukt und/oder gegebenenfalls vorhandene inerte Stoffe ganz oder teilweise in flüssiger Phase durch das Katalysatorbett leiten. Bevorzugt ist das Gasphase-Verfahren, bei dem außer dem Katalysator alle anderen Komponenten in gasförmiger Form durch den Reaktionsraum geführt werden.

Als Wasserstoff gelangt vorzugsweise Wasserstoff üblicher technischer Qualität zum Einsatz, beispielsweise Wasserstoff mit einer Reinheit von über 80 Vol.-%, vorzugsweise Wasserstoff mit einer Reinheit von über 95 Vol.-% und besonders bevorzugt Wasserstoff mit einer Reinheit von über 97 Vol.-%. Als Verunreinigungen können im Wasserstoff beispielsweise bis zu 20 Vol.-%, vorzugsweise bis zu 5 Vol.-%, besonders bevorzugt bis zu 3 Vol.-% Stickstoff, Methan, Kohlendioxid und/oder Wasserdampf vorhanden sein.

Die Strömungsgeschwindigkeit der mobilen Phase gegenüber der stationären Phase kann man beispielsweise so wählen, daß sich Katalysatorbelastungen im Bereich von 10 bis 20.000 g mobile Phase pro Liter Katalysator und pro Stunde ergeben. Bevorzugt sind Katalysatorbelastungen im Bereich 50 bis 800 g mobile Phase pro Liter Katalysator und pro Stunde.

Das nach der Durchführung des erfindungsgemäßen Verfahrens vorliegende Gemisch kann man beispielsweise aufarbeiten, indem man es z.B. auf eine Temperatur abkühlt, bei der das dechlorierte und/oder debromierte Produkt weitgehend kondensiert, z.B. auf eine Temperatur im Bereich 0 bis 50°C. Die dann verbleibende Gasphase kann man einer alkalischen Wäsche, z.B. mit Natronlauge, unterziehen, um darin enthaltenen Halogenwasserstoff zu entfernen. Den dann noch in der Gasphase zurückbleibenden überschüssigen Wasserstoff kann man in den Reaktor zurückführen. Die abgetrennte kondensierte Phase enthält im Wesentlichen das dechlorierte und/oder debromierte Produkt in technischem Reinheitsgrad, gegebenenfalls zusammen mit eingesetztem Lösungsmittel. Um reine Produkte zu erhalten, kann man die kondensierte Phase z.B. mit einem organischen Lösungsmittel aufnehmen, die Lösung mit verdünnter Natronlauge waschen und anschließend fraktioniert destillieren.

Im allgemeinen enthält das Reaktionsprodukt des erfindungsgemäßen Verfahrens keine oder nur ganz geringe Mengen an aromatischen Verbindungen, die noch Chlor oder Brom enthalten. Beim Einsatz von Verbindungen in das erfindungsgemäße Verfahren, die mehrere Chlor- und/oder Bromatome enthalten, z.B. beim Einsatz von Verbindungen der Formel (I) mit l = 2, 3 oder 4, ist es nur in speziellen Fällen möglich, die Chlor- und/oder Bromatome in Stufen nacheinander abzuspalten.

Es ist möglich, daß beim erfindungsgemäßen Verfahren nicht nur Chlor- und/oder Bromatome abgespalten werden, sondern auch an anderen Molekülteilen Reaktionen mit Wasserstoff stattfinden. Enthält die eingesetzte aromatische Verbindung z.B. eine CHO-, CH₂OH-, CH₂NH₂- oder CN- Gruppe, so bildet sich daraus im allgemeinen eine CH₃- Gruppe. Entsprechend entsteht aus einer NO₂-Gruppe eine NH₂-Gruppe. Ein zusätzlicher Wasserstoffverbrauch für derartige Reaktionen ist gegebenenfalls bei der Dosierung des Wasserstoffs zu berücksichtigen.

Mit dem erfindungsgemäßen Verfahren kann man chlor- und bromfreie aromatische Verbindungen, insbesondere solche der Formel (II) herstellen 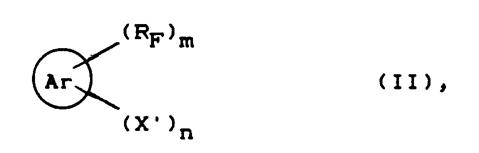
bei der
- Ar, R_{F}, m und n: die bei Formel (I) angegebene Bedeutung haben,
- X': für C₁- bis C₄-Alkyl, fluoriertes C₁- bis C₄-Alkyl, C₁- bis C₄-Alkoxy, COOC₁- bis C₄-Alkyl oder NH₂ steht,
wobei
- m+n: mindestens 1 und höchstens der Anzahl möglicher Valenzen an Ar minus 1 entspricht.

Erfindungsgemäß können aromatische, fluorhaltige, jedoch chlor- und bromfreie Verbindungen in deutlich besseren Selektivitäten und Ausbeuten erhalten werden, als bei bekannten Verfahren. Im allgemeinen liegen beim erfindungsgemäßen Verfahren die Umsätze und Selektivitäten über 90 %, die Umsätze häufig über 95 %. Dies ist aus mehreren Gründen überraschend. Zum einen erfordert das Arbeiten in der Gas- oder Rieselphase i. a. höhere Temperaturen, und höhere Temperaturen bedeuten i. a. eine Verschlechterung der Selektivität. Zum anderen besteht insbesondere bei höheren Temperaturen die Gefahr der Abspaltung von Fluorwasserstoff und damit die Gefahr der Bildung niederfluorierter Produkte. Schließlich kann grundsätzlich auch der aromatische Kern hydriert werden. Alles dies tritt überraschenderweise nicht oder nur in sehr untergeordnetem Maße auf.

Die erfindungsgemäß herstellbaren Produkte können beispielsweise als Zwischenprodukte für Farbstoffe, Pflanzenschutzmittel und Arzneimittel gebraucht werden. Beispielsweise sind 3-Alkylbenzotrifluoride wichtige Zwischenprodukte für Diazofarbstoffe (siehe DE-OS 32 01 112).

### Beispiele

### Allgemeine Arbeitsvorschrift

Ein senkrecht stehendes, beheizbares Quarzrohr mit Wasserstoffzuleitung wurde mit 200 ml 0,5 Gew.-% Palladium auf Aktivkohle enthaltendem Katalysator gefüllt. Das Zudosieren des Edukts erfolgte über eine Dosierpumpe, das Zudosieren des Wasserstoffs mittels eines Rotameters. Nach Spülung und Trocknung des Edelmetallkontaktes mit Stickstoff bei Normaldruck wurden 0,2 bis 0,25 Mol pro Stunde des Edukts und 1 Mol Wasserstoff pro Chloratom des Edukts, bei der in der Tabelle angegebenen Temperatur in das Quarzrohr eingeleitet. Der Wasserstoff hatte eine Reinheit von über 97 %. Das entsprach einer Katalysatorbelastung von 210 bis 250 g/l und pro Stunde. Bei Raumtemperatur feste Edukte wurden in Form von 20 bis 50 gew.-%igen Lösungen in Toluol eingesetzt. Das das Quarzrohr verlassende Reaktionsgemisch wurde bei 0 bis 20°C kondensiert. Die kondensierten Anteile wurde mit Wasser gewaschen, über Natriumsulfat getrocknet und anschließend gaschromatographisch analysiert. In einigen Fällen wurden die kondensierten Anteile nach dem Waschen und Trocknen destillativ aufgearbeitet.

Im einzelnen wurden folgende Umsetzungen durchgeführt:

### Beispiel 1

Aus 1-Trifluormethyl-monochlornaphthalin (Isomerengemisch) wurde bei 210°C 1-Trifluormethylnaphthalin bei einem Umsatz von 100 % mit einer Selektivität von 94,6 % erhalten.

### Beispiel 2

Aus Bis-Trifluormethyl-monochlornaphthalin (Isomerengemisch) wurde bei 210°C Bis-Trifluormethylnaphthalin (Isomerengemisch) bei einem Umsatz von 98,3 % mit einer Selektivität von 93,8 % erhalten.

### Beispiel 3

Aus 2,3-Difluor-6-chlor-ethoxybenzol wurde bei 250°C 2,3-Difluor-ethoxybenzol bei einem Umsatz von 85,3 % mit einer Selektivität von 96,2 % erhalten.

### Beispiel 4

Aus 3,4-Difluor-5-chlor-trifluormethylbenzol wurde bei 210°C 3,4-Difluor-trifluormethylbenzol bei einem Umsatz von 99,0 % mit einer Selektivität von 98 % erhalten.

### Beispiel 5

Aus 3,4-Difluor-6-chlor-trifluormethylbenzol wurde bei 230°C 3,4-Difluor-trifluormethylbenzol bei einem Umsatz von 99,6 % mit einer Selektivität von 98,8 % erhalten.

### Beispiel 6

Aus 2,3,4-Trifluor-5-chlor-trifluormethylbenzol wurde bei 200°C 2,3,4-Trifluor-trifluormethylbenzol bei einem Umsatz von 98,9 % mit einer Selektivität von 91,2 % erhalten.

### Beispiel 7

Aus 2,4-Dichlor-trifluormethoxybenzol wurde bei 230°C Trifluormethoxybenzol bei einem Umsatz von 100 % mit einer Selektivität von 91,4 % erhalten.

### Beispiel 8

Aus 2,4-Dichlor-6-trifluormethyl-trifluormethoxybenzol wurde bei 230°C 2-Trifluormethyl-trifluormethoxybenzol bei einem Umsatz von 100 % mit einer Selektivität von 92,2 % erhalten.

### Beispiel 9

Aus 3-Trifluormethyl-4-chlor-toluol wurde bei 240°C 3-Trifluormethyltoluol bei einem Umsatz von 99,4 % mit einer Selektivität von 98,1 % erhalten.

### Beispiel 10

Aus 3-Trifluormethyl-4,6-dichlortoluol wurde unter Einsatz von 10 Mol Wasserstoff pro Mol Edukt bei 220°C 3-Trifluormethyltoluol bei einem Umsatz von 100 % mit einer Selektivität von 97,4 % erhalten.

### Beispiel 11

Aus 1,3,5-Trifluor-2,4,6-trichlorbenzol wurde unter Einsatz von 10 Mol Wasserstoff pro Mol Edukt 1,3,5-Trifluorbenzol bei einem Umsatz von 100 % mit einer Selektivität von 93,8 % erhalten.

### Beispiel 12

Aus 6-Brom-2,2,3,3-tetrafluor-1,4-benzodioxen wurde bei 210°C 2,2,3,3-Tetrafluor-1,4-benzodioxen bei einem Umsatz von 100 % mit einer Selektivität von 98,4 % erhalten.

### Beispiel 13

Aus 5-Chlor-4-fluor-2-trifluormethylbenzaldehyd wurde bei 210°C bei einem Umsatz von 96,7 % 4-Fluor-2-trifluormethyl-toluol mit einer Selektivität von 72,4 % und 5-Chlor-4-fluor-2-trifluormethyltoluol mit einer Selektivität von 16,3 % erhalten.

### Beispiel 14

3-Chlor-tetrafluorbenzonitril wurde bei 240°C umgesetzt. Die Reaktionsprodukte wurden in 300 ml Wasser aufgefangen und nach Ende der Reaktion mit 40 gew.-%iger wäßriger Natronlauge alkalisch gestellt. Das organische Material wurde mit Methyl-tert.-butylether extrahiert, analytisch untersucht und anschließend destillativ aufgearbeitet. Mit einer Ausbeute von 77,3 % wurde 2,3,4,6-Tetrafluor- toluol mit einem Siedepunkt von 108°C isoliert.

### Beispiel 15

26,5 g 4-Chlor-3-amino-N,N-bistrifluormethyl-aminobenzol wurden in 50 g Toluol gelöst und bei 350°C umgesetzt.

Die Reaktionsprodukte wurden in 150 ml Wasser eingeleitet. Das erhaltene zweiphasige Gemisch wurde mit 20 gew.-%iger wäßriger Natronlauge alkalisch gestellt, und die Toluolphase abgetrennt. Aus dieser wurden durch Destillation 10,3 g 3-Amino-N,N-bistrifluormethyl-aminobenzol mit einem Siedepunkt von 76 bis 78°C bei 20 mbar erhalten.

### Beispiel 16

500 g 3-Chlor-4-trifluormethoxy-toluol wurde bei 250°C umgesetzt. Das Reaktionsprodukt wurde in einer gekühlten Vorlage aufgefangen und Chlorwasserstoff über einen Intensiv-Kühler einer Neutralisation zugeleitet. Aus der gekühlten Vorlage wurde durch Destillation 4-Trifluormethoxybenzol mit einer Ausbeute von 86,4 % isoliert. Der Siedepunkt des Produktes betrug 129 bis 131°C.

### Beispiel 17

Aus 4-Fluorchlorbenzol wurde bei 200°C Fluorbenzol bei einem Umsatz von 89 % und mit einer Selektivität von 88 % erhalten.

### Beispiel 18

Es wurde gearbeitet wie in Beispiel 17, jedoch enthielt der verwendete Katalysator Aluminiumoxid als Trägermaterial. Der Umsatz lag hierbei bei 100 %, die Selektivität bei 96,6 %.

## Patentansprüche

1. Verfahren zur selektiven Dechlorierung und/oder Debromierung von Fluor und Chlor und/oder Brom enthaltenden aromatischen Verbindungen mit Wasserstoff in Gegenwart von Katalysatoren, dadurch gekennzeichnet, daß sich der Katalysator in stationärer Phase und die Fluor und Chlor und/oder Brom enthaltende Verbindung in mobiler Phase befinden.

2. Verfahren nach Anspruch 1, dadurch gekennzeichnet, daß man als Fluor und Chlor und/oder Brom enthaltende aromatische Verbindung eine Verbindung der Formel (I) einsetzt 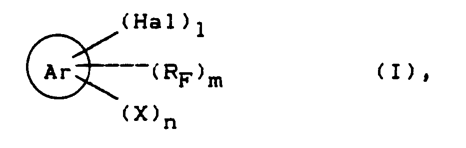 in der
Ar für einen aromatischen Rest mit 6 bis 10 C-Atomen,
Hal unabhängig voneinander für Chlor oder Brom,
R_{F} unabhängig voneinander für Fluor oder einen Fluor enthaltenden Rest mit 1 bis 4 C-Atomen und 1 bis 6 Fluoratomen,
X für C₁- bis C₄-Alkyl, fluoriertes C₁- bis C₄-Alkyl, C₁- bis C₄-Alkoxy, CHO, CH₂OH, CN, COO-C₁- bis C₄-Alkyl, NH₂, CH₂NH₂ oder NO₂,
l für 1, 2, 3 oder 4,
m für 1, 2, 3 oder 4 und
n für Null oder 1 stehen,
wobei l + m + n mindestens 2 und höchstens der Anzahl möglicher Valenzen an Ar entspricht.

3. Verfahren nach Ansprüchen 1 und 2, dadurch gekennzeichnet, daß man auf Äquivalent Chlor- und Brom-Atome, die in der jeweils eingesetzten Fluor und Chlor und/oder Brom enthaltenden aromatischen Verbindung enthalten sind, 1 bis 50 Mol Wasserstoff einsetzt.

4. Verfahren nach Ansprüchen 1 bis 3, dadurch gekennzeichnet, daß man Trägerkatalysatoren einsetzt, die Übergangsmetalle als katalytisch aktive Komponente enthalten.

5. Verfahren nach Ansprüchen 1 bis 4, dadurch gekennzeichnet, daß man es bei Drucken im Bereich 0,1 bis 20 bar durchführt.

6. Verfahren nach Ansprüchen 1 bis 5, dadurch gekennzeichnet, daß man es bei Temperaturen über 100°C durchführt.

7. Verfahren nach Ansprüchen 1 bis 6, dadurch gekennzeichnet, daß man den Wasserstoff im Gemisch mit dem Edukt gasförmig über fest angeordneten Katalysator leitet.

8. Verfahren nach Ansprüchen 1 bis 6, dadurch gekennzeichnet, daß man Wasserstoff gasförmig und das Edukt ganz oder teilweise in flüssiger Phase über einen fest angeordneten Katalysator leitet.

9. Verfahren nach Ansprüchen 1 bis 8, dadurch gekennzeichnet, daß man die Strömungsgeschwindigkeit der mobilen Phase gegenüber der stationären Phase so wählt, daß sich Katalysatorbelastungen in Bereich von 10 bis 20.000 g mobile Phase pro Liter Katalysator und pro Stunde ergeben.

## Claims

1. Process for the selective dechlorination and/or debromination of fluorine- and chlorine- and/or bromine-containing aromatic compounds with hydrogen in the presence of catalysts, characterised in that the catalyst is present in stationary phase and the fluorine- and chlorine- and/or bromine-containing compound in mobile phase.

2. Process according to Claim 1, characterised in that the fluorine- and chlorine- and/or bromine-containing aromatic compound used is a compound of the formula (I) 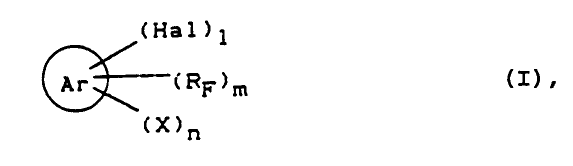 in which
Ar represents an aromatic radical having 6 to 10 C atoms,
Hal, independently of one another, represent chlorine or bromine,
R_{F}, independently of one another, represent fluorine or a fluorine-containing radical having 1 to 4 C atoms and 1 to 6 fluorine atoms,
X represents C₁- to C₄-alkyl, fluorinated C₁- to C₄-alkyl, C₁- to C₄-alkoxy, CHO, CH₂OH, CN, COO-C₁-to C₄-alkyl, NH₂, CH₂NH₂ or NO₂,
l represents 1, 2, 3 or 4,
m represents 1, 2, 3 or 4 and
n represents zero or 1,
where l + m + n corresponds to at least 2 and at most to the number of possible valencies on Ar.

3. Process according to Claims 1 and 2, characterised in that 1 to 50 mol of hydrogen are used per equivalent of chlorine and bromine atoms contained in the fluorine- and chlorine- and/or bromine-containing aromatic compound used in each case.

4. Process according to Claims 1 to 3, characterised in that the catalysts used are supported catalysts containing transition metals as the catalytically active component.

5. Process according to Claims 1 to 4, characterised in that it is carried out at pressures in the range from 0.1 to 20 bar.

6. Process according to Claims 1 to 5, characterised in that it is carried out at temperatures above 100°C.

7. Process according to Claims 1 to 6, characterised in that the hydrogen is passed over the fixed-bed catalyst in a mixture with the educt in gaseous form.

8. Process according to Claims 1 to 6, characterised in that the hydrogen is passed over a fixed-bed catalyst in gaseous form and the educt completely or partially in liquid phase.

9. Process according to Claims 1 to 8, characterised in that the flow rate of the mobile phase with respect to the stationary phase is selected such that the resulting catalyst space velocities are in the range from 10 to 20,000 g of mobile phase per litre of catalyst per hour.

## Revendications

1. Procédé pour déchlorer et/ou débromer sélectivement des composés aromatiques contenant du fluor et du chlore et/ou du brome à l'aide d'hydrogène en présence de catalyseurs, caractérisé en ce que le catalyseur est en phase stationnaire et le composé contenant du fluor et du chlore et/ou du brome en phase mobile.

2. Procédé selon la revendication 1, caractérisé en ce que le composé aromatique contenant du fluor et du chlore et/ou du brome et qui est mis en oeuvre répond à la formule (I) : 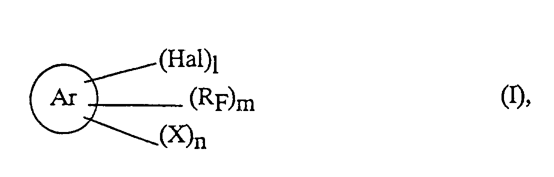 dans laquelle
Ar représente un radical aromatique en C₆-C₁₀,
les symboles Hal représentent chacun, indépendamment les uns des autres, le chlore ou le brome,
les symboles R_{F} représentent chacun, indépendamment les uns des autres, le fluor ou un radical fluoré contenant 1 à 4 atomes de carbone et 1 à 6 atomes de fluor,
les symboles X représentent chacun un groupe alkyle en C₁-C₄, un groupe alkyle fluoré en C₁-C₄, un groupe alcoxy en C₁-C₄, CHO, CH₂OH, CN, COO-alkyle en C₁-C₄, NH₂, CH₂NH₂ ou NO₂,
l est égal à 1, 2, 3 ou 4,
m est égal à 1, 2, 3 ou 4 et
n est égal à 0 ou 1,
la somme l + m + n étant égale au minimum à 2 et au maximum au nombre des valences possibles de Ar.

3. Procédé selon les revendications 1 et 2, caractérisé en ce que l'on met en oeuvre de 1 à 50 moles d'hydrogène par équivalent d'atomes de chlore et de brome contenu dans le composé aromatique contenant du fluor et du chlore et/ou du brome mis en oeuvre dans chaque cas.

4. Procédé selon les revendications 1 à 3, caractérisé en ce que l'on utilise des catalyseurs sur support dont les composants catalytiques actifs sont des métaux de transition.

5. Procédé selon les revendications 1 à 4, caractérisé en ce que l'on opère à des pressions dans l'intervalle de 0,1 à 20 bars.

6. Procédé selon la revendication 1 à 5, caractérisé en ce que l'on opère à des températures supérieures à 100°C.

7. Procédé selon les revendications 1 à 6, caractérisé en ce que l'on envoie l'hydrogène, en mélange avec le produit de départ à l'état gazeux, sur le catalyseur en disposition fixe.

8. Procédé selon les revendications 1 à 6, caractérisé en ce que l'on envoie l'hydrogène à l'état gazeux et le produit de départ en totalité ou en partie en phase liquide, sur un catalyseur en disposition fixe.

9. Procédé selon les revendications 1 à 8, caractérisé en ce que l'on règle la vitesse d'écoulement de la phase mobile par rapport à la phase stationnaire en sorte que la charge du catalyseur se situe dans l'intervalle de 10 à 20 000 g de la phase mobile par litre du catalyseur et par heure.
